**Europäisches Patentamt**

**European Patent Office**

**Office européen des brevets**

(11) Veröffentlichungsnummer : **0 403 993 B1**

(12) **EUROPÄISCHE PATENTSCHRIFT**

(45) Veröffentlichungstag der Patentschrift :
23.09.92 Patentblatt 92/39

(51) Int. Cl.⁵ : **C07D 307/62**

(21) Anmeldenummer : **90111414.0**

(22) Anmeldetag : **18.06.90**

(54) **Verfahren zur Herstellung von Natrium- oder Kalium-L-Ascorbat.**

(30) Priorität : **20.06.89 CH 2377/89**

(43) Veröffentlichungstag der Anmeldung :
**27.12.90 Patentblatt 90/52**

(45) Bekanntmachung des Hinweises auf die
Patenterteilung :
**23.09.92 Patentblatt 92/39**

(84) Benannte Vertragsstaaten :
**AT BE CH DE DK ES FR GB GR IT LI LU NL SE**

(56) Entgegenhaltungen :
**DE-B- 1 468 267**
**US-A- 2 179 978**
**Helv. Chim. Acta 17, 311, 1934.**

(73) Patentinhaber : **ENCO ENGINEERING CHUR
AG
Sägenstrasse 97
CH-7000 Chur (CH)**

(72) Erfinder : **Keglevic, Tomislav
Jubiläumstrasse 16
A-2352 Gumpoldskirchen (AT)**
Erfinder : **Klein, Christoph
Mutschellenstrasse 14
CH-8002 Zürich (CH)**

(74) Vertreter : **Lauer, Joachim, Dr.
Hug Interlizenz AG Austrasse 44 Postfach
CH-8045 Zürich (CH)**

EP 0 403 993 B1

## Beschreibung

Die vorliegende Erfindung betrifft ein Verfahren zur Herstellung von Natrium- oder Kalium-L-Ascorbat unter Verwendung von 2-Keto-L-Gulonsäure, bei welchem 2-Keto-L-Gulonsäure-Methylester gebildet und durch Zugabe von Bicarbonat laktonisiert wird.

## Stand der Technik

Natrium-L-Ascorbat fällt in der industriellen Praxis u.a. bei der Herstellung von L-Ascorbinsäure als Zwischenprodukt bei dem üblicherweise verwendeten mehr oder weniger modifizierten sogenannten Reichstein-Verfahren (T. Reichstein, A. Grüssner, Helv. Chim. Acta l7, 3ll, l934) an. Bei diesem Verfahren tritt auf einer früheren Reaktionsstufe als Zwischenprodukt Di-iso-Propyliden-2-Keto-L-Gulonsäure-Monohydrat auf. Dessen Methylester wird durch Laktonisierung mittels Zugabe von Natrium-Bikarbonat in das Natrium-L-Ascorbat überführt, aus dem schliesslich die L-Ascorbinsäure gewonnen wird.

Grundsätzlich limitiert das Veresterungsgleichgewicht die Ausbeute. Beim Reichstein-Verfahren wird zur Erzielung eines hohen Veresterungsgrades und damit einer befriedigenden Ausbeute üblicherweise eine lange Kochzeit gewählt. Diese wirkt sich jedoch zu Lasten der Reinheit aus. Sowohl die 2-L-Keto-Gulonsäure als auch ihr Methylester sind thermisch nicht sehr stabil. Sie können bereits bei den erforderlichen Reaktionstemperaturen zerfallen. Die Zerfallsprodukte sowie die nicht umgesetzte 2-Keto-L-Gulonsäure stellen im Endprodukt Verunreinigungen dar, deren Entfernung einen erheblichen Aufwand erfordert. Zur Herstellung von Natrium-L-Ascorbat als Endprodukt in für pharmazeutische Zwecke ausreichender Reinheit ist das Reichstein-Verfahren daher nicht geeignet oder zumindest nicht wirtschaftlich.

Bei der fermentativen Herstellung von 2-Keto-L-Gulonsäure-Monohydrat tritt zudem das Problem Entfernung der löslichen Proteine aus dem Fermentationsprodukt auf. Diese Proteine wirken sich ebenfalls als Verunreinigungen im Endprodukt aus. Sie sind sowohl durch Kristallisation als auch durch Ionen-Austauscher nur unter beträchtlichem, unwirtschaftlichem Aufwand und unter Produktverlust zu separieren.

Zur Herstellung von ausreichend reinem Natrium- oder Kalium--L-Ascorbat wird im Hinblick auf die vorstehend angeführten Probleme üblicherweise L-Ascorbinsäure von genügender Reinheit als Ausgangsmaterial verwendet.

## Darstellung der Erfindung

Es ist Aufgabe der vorliegenden Erfindung, ein Verfahren zur Herstellung von Natrium- oder von Kalium-L-Ascorbat unter Verwendung von 2-Keto-L-Gulonsäure anzugeben, das es erlaubt, das gewünschte Endprodukt mit ausreichender Reinheit in wirtschaftlicher Weise herzustellen.

Diese sowie weitere Aufgaben werden gemäss der vorliegenden Erfindung gelöst durch ein Verfahren, wie es im Patentanspruch I gekennzeichnet ist.

Bei dem erfindungsgemässen Verfahren wird demnach insbesondere die Veresterung der 2-Keto-L-Gulonsäure mit Methanol lediglich partiell, das heisst nicht bis zum Erreichen des Veresterungsgleichgewichts durchgeführt. Das erfindungsgemässe Verfahren benötigt daher nur sehr kurze Reaktionszeiten in der Veresterung, bedingt durch die Ausnutzung des Bereichs der grossen Reaktionsgeschwindigkeiten und durch das Ausschalten des Bereichs kleiner Reaktionsgeschwindigkeiten. Damit verbunden ist in vorteilhafter Weise auch ein bedeutend geringerer Anfall von Zersetzungsprodukten.

Weiter wird bei dem erfindungsgemässen Verfahren in einem Zwischenschritt durch Zugabe, je nach gewünschtem Endprodukt, von Natrium-Bicarbonat bzw. von Kalium-Bicarbonat in einer im wesentlichen zur Neutralisierung der Esterlösung gerade ausreichenden Menge, nicht veresterte 2-Keto-L-Gulonsäure sowie vorhandene Verunreinigungen aus der Lösung ausgefällt und abgetrennt. Erst danach wird durch Zugabe von weiterem Bicarbonat die Lactonisierung des gebildeten 2-Keto-L-Gulonsäure-Methylesters, welche im basischen Bereich erfolgt, ermöglicht.

Das erfindungsgemässe Verfahren ist ausgezeichnet durch eine hohe Reinheit der Esterlösung. Nicht umgesetzte 2-Keto-L-Gulonsäure und Verunreinigungen wie die vorgenannten Zersetzungsprodukte sowie auch die eingangs erwähnten lösliche Proteine, welche aus der fermentativen Hertstellung des 2-Keto--L-Gulonsäure-Monohydrats stammen, werden in dem genannten Zwischenschritt ausgefällt und abgetrennt.

Das erfindungsgemässe Verfahren ermöglicht die direkte Herstellung von pharmazeutisch reinem Natrium- oder Kalium-L-Ascorbat im Ablauf der L-Ascorbinsäure-Synthese.

Schliesslich ermöglicht das erfindungsgemässe Verfahren durch die kurzen Reaktionszeiten auch noch eine wirtschaftliche kontinuierliche Herstellung von 2-Keto-L-Gulonsäure-Methylester.

Vorteilhafte und bevorzugte Weiterbildungen des erfindungsgemässen Verfahrens sind in den abhängigen Ansprüchen gekennzeichnet.

Zur Gewinnung des 2-Keto-L-Gulonsäure-Methylesters kann eine Mischung aus 2-Keto-Gulonsäure-Monohydrat und Natrium-2-Keto-Gulonat verwendet werden, wobei der Anteil des Natriumsalzes bis zu 50 % betragen kann. Die Mischung aus 50% Monodydrat und 50% Salz ist stabiler als das reine Monohydrat. Im Falle eines hohen Salzanteils muss das Salz zunächst in die Säure umgewandelt werden, was durch Zugabe einer ausreichenden Menge von konzentrierter Schwefelsäure erfolgt. Das dabei entstehende Sulfat wird vorzugsweise vor der Bikarbonatzugabe gesondert abgetrennt. Diese Antrennung kann mit Vorteil auch noch vor der Zugabe der die Veresterung katalysierenden Menge an konzentrierter Schwefelsäure erfolgen. Sofern der Anteil des Natriumsalzes in der eingesetzten Mischung nicht zu hoch ist und nur wenige % beträgt kann auf das gesonderte Abtrennen des Sulfates auch vezichtet werden. Es wird dann zusammen mit dem durch die Bikarbonatzugabe verursachten Niederschlag entfernt.

Es genügt, die Veresterung lediglich bis zu einem Grad von 87 - 9l % zu treiben.

Vorzugsweise wird zur Veresterung eine Temperatur im Bereich zwischen 60 - 75° C eingestellt.

Die Veresterung in Gegenwart der konzentrierten Schwefelsäure in katalytischer Menge zur Reaktionsbeschleunigung, wird vorzugsweise innerhalb von l5 bis 90 Minuten, je nach dem Grad der Verunreinigung des Ausgangsproduktes, durchgeführt.

Die zum Ausfällen des Niederschlags erforderliche Menge Bicarbonat muss nicht nur zur Neutralisierung der noch vorhandenen 2-Keto-L-Gulonsäure sondern zusätzlich noch zur Neutralisierung der eingesetzten Schwefelsäure ausreichend bemessen sein.

Vorzugsweise wird das Bicarbonat in geringem Überschuss (über die zur Neutralisierung an sich exakt erforderliche Menge) von l - 2 % zur Veresterungslösung zugesetzt. Während und nach der Zugabe des Bicarbonats wird, vorzugsweise bei einer gleichbleibenden Temperatur von etwa 60° C, eine halbe bis zwei Stunden gerührt.

Die Abtrennung des Niederschlags, erfolgt vorzugsweise durch Filtrieren, doch sind grundsätzlich alle Fest-/Flüssig-Trennmethoden anwendbar.

Das zur Laktonisierung erforderliche Natrium- oder Kalium-Bicarbonat kann direkt zu der nach der Abtrennung des Niederschlags erhaltenen Esterlösung hinzugefügt werden.

Zur Herstellung von hochreinem Ascorbat kann jedoch auch folgender Weg beschritten werden: Die nach dem Abtrennen des Niederschlags erhaltene Esterlösung wird (auf etwa 0 °C) abgekühlt und dabei in Reinform (teilweise) ausfallender Ester, vorzugsweise wieder durch Filtrieren, abgetrennt. Der in Reinform erhaltene Ester wird in Methanol gelöst und das Verfahren mit dieser Esterlösung weitergeführt.

Das Verfahren kann jedoch auch mit der Mutterflüssigkeit weitergeführt werden, aus der der Methylester durch das Abkühlen (teilweise) ausgefallen ist, da diese immer noch genügend Methylester enthält. Das auf diesem Weg erhaltene Ascorbat ist allerdings unreiner, für gewisse Anwendungszwecke aber immer noch brauchbar.

Zur Laktonisierung des 2-Keto-L-Gulonsäure-Methylesters, wird die ihn enthaltene Lösung nach der Zugabe des weiteren Natrium- oder Kalium-Bicarbonats vorzugsweise während nur 30 bis l80 Minuten bei 60 - 67° C gekocht.

Das in Suspension erhaltene Natrium- oder Kalium-L-Ascorbat wird vorzugsweise wieder durch Filtrieren abgetrennt.

Das abgetrennte Ascorbat kann mit Vorteil auch noch mit Methanol gewaschen werden.

Die erreichbare Ausbeute lässt sich in vorteilhafter Weise dadurch steigern, dass der in dem vorgenannten Zwischenschritt abgetrennte Niederschlag als Ausgangsstoff an Stelle von frischem 2-Keto-L-Gulonsäure-Monohydrat wiederverwendet wird. Der abgetrennte Niederschlag, der je nach dem erreichten Veresterungsgrad noch 3 - l0 % des unveresterten Ausgangsproduktes enthält, wird an Stelle von frischem 2-Keto-L-Gulonsäure-Monohydrat, vorzugsweise in einem separaten Lösungsansatz, mit Methanol gelöst und in gleicher Weise, wie vorstehend beschrieben, der Veresterung, Fällung und Abtrennung des Niederschlags unterworfen. Der dabei erneut anfallende Niederschlag wird verworfen. Er enthält nur noch unwesentliche Mengen des Ausgangsproduktes, die unerwünschten Verunreinigungen jedoch in relativ hoher Konzentration.

Die verbleibende Flüssigkeit, welche den 2-Keto-L-Gulonsäure-Methylester enthält, wird nachfolgend entweder der aus dem ursprünglichen Ansatz nach dem Abtrennen des Niederschlags verbliebenen gleichartigen Flüssigkeit zugesetzt und damit in das ursprüngliche Verfahren wieder eingeführt, oder weiter separat, jedoch in analoger Weise, zum Ascorbat aufgearbeitet.

Den aus der ursprünglichen Esterlösung (dem primären Lösungsansatz) abgetrennten Niederschlag nicht separat, sondern wieder zusammen mit frischem 2-Keto-L-Gulonsäure-Monohydrat anzusetzen ist möglich aber nicht so vorteilhaft. Der Gehalt an Verunreinigungen im primären Lösungsansatz steigt dadurch an. Auch wird die Filtrierbarkeit des primären Lösungsansatzes schlechter und damit zeitraubender und teurer.

Nachstehend werden einige Beispiele für das erfindungsgemässe Verfahren angegeben, wobei auch auf das beigefügte Schema Bezug genommen wird. Die Beispiele beziehen sich sämtlich auf die Herstellung von Natrium-L-Ascorbat, sind jedoch in gleicher Weise auch auf Kalium-L-Ascorbat anwendbar, sofern statt der angegebenen Mengen in Gramm bzw. ccm die entsprechenden Mole eingesetzt werden.

Beispiel I

l00 g 2-Keto-Gulonsäure-Monohydrat, 98.3 %-ig, wird in 300 ccm Methanol (max. 0,2 % $H_2O$) mit 4.5 ccm $H_2SO_4$ (98 %) gelöst und während 30 Minuten bei 67 - 68° C verestert (Veresterung A). Anschliessend wird der Lösung bei 60° C l2,5 g Natrium-Bicarbonat (99 %) zudosiert und zwei Stunden bei gleichbleibender Temperatur gerührt (Fällen A). Der gebildete Niederschlag wird abfiltriert (Filtration A/I). Das Filtrat (Filtrat A) wird anschliessend während zwei Stunden mit weiterem Bicarbonat laktonisiert (Laktonisierung A). Das entstehende Ascorbat (Produkt A) wird schliesslich durch Filtration (Filtration A/II) isoliert.

In einem Testversuch konnten im Filtrat (Filtrat A) durch Dünnschichtchromatographie keine Spuren von 2-Ketogulonat nachgewiesen werden, wohl aber im Filterrückstand (Filterrückstand A). Dieser wies ein Trockengewicht von l0,7 g auf. Das erhaltene Ascorbat (Produkt A) wies ein Trockengewicht von 88,35 g auf, was 89,82 % der theoretisch möglichen Ausbeute entspricht. Die Reinheit des erhaltenen Ascorbats konnte durch Jodtitrierung zu 96,9 % und durch GC-Bestimmung zu 99,87 % bestimmt werden.

Der Filterrückstand (l0,7 g) (Filterrückstand A) wird in einem parallelen Lösungsansatz mit 24 ccm Methanol unter Zusatz von l,3 ccm $H_2SO_4$ bei 67 - 68° C während 30 Minuten behandelt (Veresterung B). Anschliessend wird l,3 g Natrium-Bicarbonat zudosiert und eine Stunde bei 50° C gerührt (Fällen B). Der gebildete Niederschlag (6,7 g) wird abfiltriert (Filtration B/I), der Filterrückstand (Filterrückstand B) verworfen. Das Filtrat (Filtrat B) wird anschliessend während drei Stunden mit Bicarbonat laktonisiert (Laktonisierung B) und das sich dabei bildende Ascorbat (Produkt B) schliesslich wieder durch Filtration (Filtration B/II) isoliert.

Im genannten Testversuch wurden auf diese Weise 4,4 g trockenes Ascorbat (Produkt B) erhalten. Dies entspricht einer Ausbeute, bezogen auf die eingesetzten l00 g Rohmaterial, von 4,62 %. Die Reinheit konnte durch Jodtitrierung zu 96,4 % und durch GC-Bestimmung zu 99 % bestimmt werden.

Die im Testversuch erzielte Gesamtausbeute betrug 94,44 % (Produkte A + B), die erzielte durchschnittliche Reinheit des Gesamtproduktes 99,8 % (GC-Bestimmung).

Beispiel 2

Der gleiche Veresterungsansatz wie im Beispiel I wird während einer Stunde bei 67 - 68° C gehalten (Veresterung A) und anschliessend mit l2,5 g Natrium-Bicarbonat während zwei Stunden bei 60° C gerührt (Fällen A).

In einem Testversuch ergab die Filtration (Filtration A/I) 9,4 g Filterrückstand (Filterrückstand A). Im Filtrat (Filtrat A) war kein 2-Ketogulonat nachweisbar. Aus dem Filtrat (Filtrat A) konnte, auf die gleiche Weise wie in Beispiel I angegeben, 88,35 g (90.83 % der Theorie) trockenes Natrium-Ascorbat (Produkt A) hergestellt werden mit einer Reinheit von 94,4 % (Jodtitration) bzw. 99,6% (GC-Bestimmung).

Der vorgenannte Filterrückstand (Filterrückstand A) wird wiederum wie im Beispiel I in 24 ccm Methanol mit l,3 ccm $H_2SO_4$ während einer Stunde verestert (Veresterung B) und die Lösung anschliessend mit l,3 g Natrium-Bicarbonat bei 60° C während einer halben Stunde behandelt (Fällen B). Der Niederschlag wird abfiltriert und der Filterrückstand (Filterrückstand B) verworfen.

Im genannten Testversuch wurde nach dem Ausfällen und Filtrieren 6,3 g Filterrückstand erhalten. Im Filtrat (Filtrat B) war kein 2-Ketogulonat mehr nachweisbar. Aus dem Filtrat (Filtrat B) konnten auf die gleiche Weise, wie im Beispiel I angegeben, 3,6 g trockenes Natrium-Ascorbat (Produkt B) entsprechend einer Ausbeute von 3,57 % der Theorie hergestellt werden und zwar mit einer Reinheit von 9l % (Jodtitration) bzw. 98,7 % (GC-Bestimmung).

Die im Testversuch erzielte Gesamtausbeute betrug 94,4 % (Produkt A + B) und die durchschnittliche Reinheit des Gesamtproduktes 99,6 % (GC-Bestimmung).

Beispiel 3

Einem Ansatz von l00 g 2-Keto-L-Gulonsäure-Monohydrat, 325 ccm Methanol und 5,5 ccm $H_2SO_4$ wird l0 g Filterrückstand, beispielsweise der Art von Beispiel I (Filterrückstand A), zugesetzt. Die Lösung wird anschliessend während einer Stunde bei 67 - 68° C verestert (Veresterung A) und nachfolgend unter den gleichen Bedingungen wie in den vorangegangenen Beispielen mit l3,5 g Natrium-Bicarbonat behandelt.

In einem Testversuch wurde nach dem Ausfällen und Filtrieren l5,8 g Filterrückstand erhalten. Aus dem Filtrat konnten durch Laktonisierung, ebenfalls wieder in gleicher Weise wie in den vorangegangenen Beispielen, 9l,64 g trockenes Natrium-Ascorbat entsprechend einer Ausbeute von 93,52 % der Theorie mit einer Reinheit von 93,7 % (Jodtitration) bzw. 99,3 % (GC-Bestimmung) herstellt werden.

Beispiel 4

l00 g 2-Keto-L-Gulonsäure-Monohydrat wird wie in Beispiel I behandelt (Veresterung A, Fällen A, Filtration A/I). Der sich ergebende Filterrückstand (Filterrückstand A) wird nachfolgend ebenfalls wie in Beispiel I weiterbehandelt (Veresterung B, Fällen B, Filtration B/I). Es ergibt sich ein zweites Filtrat (Filtrat B) sowie erneut ein Filterrückstand (Filterrückstand B). Letzterer wird verworfen. Im Unterschied zu Beispiel I werden die beiden Filtrate (Filtrat A und Filtrat B) jedoch vermischt und mit Bicarbonat über zwei Stunden gekocht.

In einem Testversuch konnten 90,l g trockenes Ascorbat entsprechend einer Ausbeute von 94,22 % gewonnen werden mit einer Reinheit von 96,0 % (Jodtitrierung) bzw. 99,7 % (GC-Bestimmung).

Beispiel 5

Eine Mischung (50g) von 2-Keto-L-Gulonsäure (5l,57%) und Natrium-2-Keto-L-Gulonat (48,43%) wird in Methanol suspendiert (l30 ml) und 4,5 ml konzentrierte Schwefelsäure zugegeben. Die Suspension wird eine Stunde unter Rühren am Rückfluss erhitzt und anschliessend filtriert zur Entfernung des entstandenen Natriumsulfats (4,77 g). Natrium-Bikarbonat (7,0g) wird zu dem Filtrat zugegeben und die Suspension eine weitere Stunde unter Rühren am Rückfluss erhitzt.

Anschliessend wird erneut gefiltert. Dabei werden 8,7 g Sediment erhalten. Zur Laktonisierung wird eine weitere Menge Natrium-Bikarbonat (l5 g) zum Filtrat zugegeben. Die Suspension wird unter Rühren für zwei Stunden am Rückfluss erhitzt. Das entstandene Natrium-Ascorbat wird angetrennt durch Filtrieren. 38,35 g trockenes Endprodukt mit einer Reinheit von 94,5% werden erhalten.

Beispiel 6

2-Keto-L-Gulonsäure-Monohydrat (300 g, l,4l5 Mol) wird in ein l000 ml Glassreaktionsgefäss gegeben und Methanol hinzugefügt (780 ml). Schwefelsäure (3 ml) wird tropfenweise zugefügt unter Rühren und die Reaktionslösung eine Stunde lang am Rückfluss erhitzt (68°C). Danach wird die genannte Lösung auf 60°C abgekühlt, 3l,2 g Natrium-Bikarbonat zugegeben und das Rühren sowie Erhitzen am Rückfluss für eine weitere Stunde fortgesetzt. Der in der Reaktionslösung enthaltene Niederschlag wird durch Filtrieren abgetrennt. Es wird 33,l9 g trockenes Sediment enthalten, das 48% der 2-Keto-L-Gulonsäure als Natriumsalz enthält. Das Filtrat wird gekühlt auf 5°C und ausfallender reiner 2-Keto-L-Gulonsäure-Methylester erneut abfiltriert und getrocknet (l90 g). 20 g des dieses reinen Esters wird in Methanol (80 ml) gelöst und Natrium-Bikarbonat (7,2 g) zugegeben. Die Suspension wird unter Rühren zwei Stunden lang am Rückfluss erhitzt. Das entstehende Ascorbat wird abfiltriert, mit l0 ml Methanol gewaschen und schliesslich getrocknet. Es resultieren l9,27 g trockenes Produkt mit einer Reinheit von 98,l5%.

Zur weiteren Erhöhung seiner Reinheit wird das erhaltene Ascorbat noch rekristallisiert. Es wird dazu in 20 ml destilliertem Wasser gelöst. Die Lösung wird filtriert und anschliessend mit 40 ml Methanol versetzt. Die entstehende Suspension wird auf 0°C heruntergekühlt und das gewünschte Produkt abfiltriert und getrocknet. l4,44 g pharmazeutisch reines Natrium-Ascorbat wird erhalten mit einer Reinheit von 99,34%.

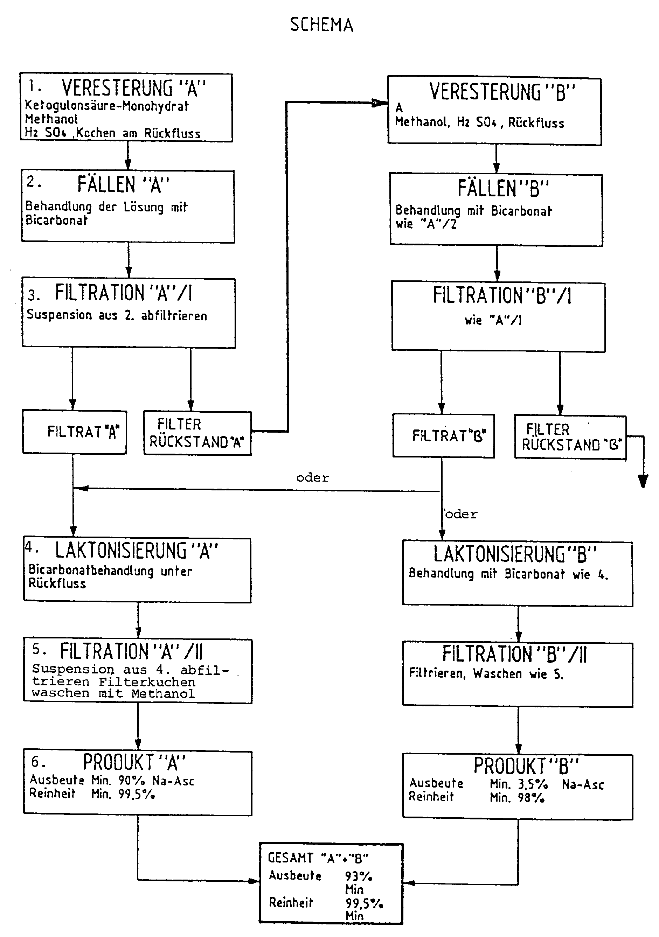

**Patentansprüche**

I. Verfahren zur Herstellung von Natrium- oder Kalium-L-Ascorbat unter Verwendung von 2-Keto-L-Gulonsäure, bei welchem 2-Keto-L-Gulonsäure-Methylester gebildet und durch Zugabe von Bicarbonat laktonisiert wird, gekennzeichnet durch folgende Verfahrensschritte:

Gewinnen des 2-Keto-L-Gulonsäure-Methylesters durch Lösen einer 2-Keto-L-Gulonsäure-Monohydrat enthaltenden Mischung in Methanol in Gegenwart von konzentrierter Schwefelsäure in zumindest katalytischer Menge;

Zugabe, je nach gewünschtem Endprodukt, von Natrium-Bicarbonat bzw. von Kalium-Bicarbonat in einer zum Ausfällen von nicht verestertem 2-Keto-L-Gulonsäure-Monohydrat sowie vorhandener Verunreinigungen aus der Lösung ausreichenden, zur Lactonisierung des 2-Keto-L-Gulonsäure-Methylesters jedoch nicht ausreichenden Menge noch vor Erreichen des Gleichgewichts der Veresterungsreaktion;

Abtrennen des Niederschlags;

Zugabe, je nach gewünschtem Endprodukt, von Natrium-Bicarbonat bzw. von Kalium-Bicarbonat zur Laktonisierung des 2-Keto-L-Gulonsäure-Methylesters; und

Abtrennen des durch die Laktonisierung entstandenen, in Suspension befindlichen Natrium-L-Ascorbats bzw. Kalium-L-Ascorbats.

2 Verfahren nach Anspruch I, dadurch gekennzeichnet, dass die genannte, 2-Keto-L-Gulonsäure-Monohydrat enthaltende Mischung weiter Natrium-2-Keto-L-Gulonat enthält und dass die gesamte Menge an konzentrierter Schwefelsäure so bemessen wird, dass sie im wesentlichen auch zur Umwandlung des Natrium-2-Keto-L-Gulonats in 2-Keto-L-Gulonsäure ausreicht.

3. Verfahren nach Anspruch 2, dadurch gekennzeichnet, dass das bei der Umwandlung des Natrium-2-Keto-L-Gulonats in 2-Keto-L-Gulonsäure anfallende Natriumsulfat vor der Bikarbonatzugabe abfiltriert wird.

4. Verfahren nach Anspruch 3, dadurch gekennzeichnet, dass zunächst im wesentlichen nur soviel konzentrierte Schwefelsäure eingesetzt wird, wie zur Umwandlung des Natrium-2-Keto-L-Gulonats in 2-Keto-L-Gulonsäure erforderlich ist und dass die die Veresterung katalysierende Menge an konzentrierter Schwefelsäure erst nach dem Abfiltrieren des entstandenen Natriumsulfats zugegeben wird.

5. Verfahren nach einem der Ansprüche I bis 4, dadurch gekennzeichnet, dass die Veresterung der 2-Ke-to-Gulonsäure mit Methanol in nur I5 bis 90 Minuten, je nach dem Grad der Verunreinigung des Ausgangsproduktes, bei 60 - 75° C bis zu einem Veresterungsgrad von 87 - 9I % durchgeführt wird.

6. Verfahren nach einem der Ansprüche I bis 4, dadurch gekennzeichnet, dass nach der erstmaligen Zugabe von Natrium-Bicarbonat bzw. von Kalium-Bicarbonat bei gleichbleibender Temperatur von vorzugsweise etwa 60 °C zum Ausfällen des Niederschlags während einer 30 - I20 Minuten gerührt wird.

7. Verfahren nach einem der Ansprüche I bis 6, dadurch gekennzeichnet, dass die nach der Abtrennung des Niederschlags verbleibende Flüssigkeit auf eine Temperatur im Bereich zwischen 0 - 5 °C abgekühlt und der dadurch ausfallende 2-Keto-L-Gulonsäure-Methylester abgetrennt wird.

8. Verfahren nach Anspruch 7, dadurch gekennzeichnet, dass das zur Laktonisierung des 2-Keto-L-Gulonsäure-Methylesters erforderliche Natrium-Bikarbonat bzw. Kalium-Bikarbonat zu der nach der Abtrennung des Niederschlags und der Abtrennung des durch die Temperaturerniedrigung ausgefallenen 2-Keto-L-Gulonsäure-Methylesters verbleibenden Flüssigkeit zugegeben wird.

9. Verfahren nach Anspruch 7, dadurch gekennzeichnet, dass der abgetrennte 2-Keto-L-Gulonsäure-Methylester erneut in Methanol gelöst wird und dass das zur Laktonisierung des 2-Keto-L-Gulonsäuremethylesters erforderliche Natrium-Bikarbonat bzw. Kalium-Bikarbonat zu dieser Lösung zugegeben wird.

I0. Verfahren nach einem der Ansprüche I bis 9, dadurch gekennzeichnet, dass die 2-Keto-L-Gulonsäure-Methylester enthaltende Flüssigkeit, zu der das weitere Natrium- oder Kalium-Bicarbonat zwecks Lacktonisierung dieses Esters zugegeben wird, nach dieser Zugabe während 30 bis I80 Minuten bei 60 - 67° C gekocht wird.

II. Verfahren nach einem der Ansprüche I bis I0, dadurch gekennzeichnet, dass der abgetrennte Niederschlag als Ausgangsstoff an Stelle von frischem 2-Keto-L-Gulonsäure-Monohydrat erneut in das Verfahren ein-geführt wird

I2. Verfahren nach Anspruch I0, dadurch gekennzeichnet, dass der abgetrennte Niederschlag an Stelle von frischem 2-Keto-L-Gulonsäure-Monohydrat in einem separaten Lösungsansatz wiederverwendet wird und dass der aus diesem Lösungsansatz ausgefällte Niederschlag verworfen wird.

## Claims

1. Process for the production of sodium or potassium L-ascorbate, using 2-keto-L-gulonic acid, in which 2-keto-L-gulonic acid is formed, and lactonized by the addition of bicarbonate, characterized by the following process steps :

   – obtaining 2-keto-L-gulonic acid methyl ester by dissolving a mixture containing 2-keto-gulonic acid monohydrate in methanol in the presence of concentrated sulphuric acid in an at least catalytic amount;

   – adding, according to the end product desired, sodium or potassium bicarbonate in an amount suffi-cient for the precipitation of unesterified 2-keto-L-gulonic acid monohydrate, and impurities present from the solution, but not sufficient for lactonizing the 2-keto-L-gulonic acid methyl ester before reaching the equilibrium of the esterification reaction;

   – separating the precipitate;

   – adding, according to the desired end product, sodium or potassium bicarbonate for lactonizing of the 2-keto-L-gulonic acid methyl ester, and separating the sodium L-ascorbate or potassium L-ascorbate resulting from the lactonization and present in the suspension.

2. Process according to claim 1, characterized in that the mixture containing 2-keto-L-gulonic acid monohy-

EP 0 403 993 B1

drate also contains sodium-2-keto-L-gulonate, and that the total amount of concentrated sulphuric acid is such that it suffices, substantially, also for the conversion of sodium 2-keto-L-gulonate into 2-keto-L-gulonic acid.

3. Process according to claim 2, characterized in that the sodium sulphate obtained in the conversion of sodium-2-keto-L-gulonate into sodium-2-keto-L-gulonic acid is filtered out before the bicarbonate is added.

4. Process according to claim 3, characterized in that, at first, only about as much concentrated sulphuric acid is added as is necessary for the conversion of sodium-2-keto-L-gulonate into 2-keto-L-gulonic acid and that the amount of concentrated sulphuric acid catalyzing the esterification is only added after filtering out the resultant sodium sulphate.

5. Process according to one of claims 1 to 4, characterized in that the esterification of 2-keto-gulonic acid with methanol is carried out in only 15 to 90 minutes, according to the degree of impurity of the raw product, at 60 to 75°C to a degree of esterification of 87-91%.

6. Process according to one of the claims 1 to 4, characterized in that after the first addition of sodium or potassium bicarbonate, the mixture is stirred at a constant temperature of preferably about 60°C for the precipitation of deposit for 30 to 120 minutes.

7. Process according to one of the claims 1 to 6, characterized in that the fluid remaining after separating the precipitate is cooled to a temperature in a range between 0 and 5°C, and the 2-keto-L-gulonic acid methyl ester precipitated in this way is separated.

8. Process according to claim 7, characterized in that the sodium or potassium bicarbonate necessary for lactonizing the 2-keto-L-gulonic acid methyl ester is added to the fluid remaining after the separation of the precipitate and the separation of the 2-keto-L-gulonic acid methyl ester precipitated by lowering the temperature.

9. Process according to claim 7, characterized in that the separated 2-keto-L-gulonic acid methyl ester is dissolved again in methanol, and that the sodium or potassium bicarbonate necessary for lactonizing the 2-keto-L-gulonic acid methyl ester is added to this solution.

10. Process according to one of the claims 1 to 9, characterized in that the fluid containing 2-keto-L-gulonic acid methyl ester, to which more sodium or potassium bicarbonate has been added in order to lactonize this ester, is boiled, after this addition, for 30 to 180 minutes at 60 to 67°C.

11. Process according to one of the claims 1 to 10, characterized in that the separate precipitate is introduced into the process again, as raw material, instead of fresh 2-keto-L-gulonic acid monohydrate.

12. Process according to claim 10, characterized in that the separated precipitate is used again, instead of fresh 2-keto-L-gulonic acid monohydrate, in a separate solution, and that the precipitate from this solution is discarded.

**Revendications**

1. Procédé pour la préparation du L-ascorbate de sodium ou de potassium au moyen de l'acide 2-céto-L-gulonique, dans lequel du 2-céto-L-gulonate de méthyle est formé et lactonisé par addition de bicarbonate, caractérisé par les étapes de processus suivantes:

obtention du 2-céto-L-gulonique de méthyle par dissolution d'un mélange contenant de l'acide 2-céto-L-gulonique monohydraté, dans du méthanol, en présence d'acide sulfurique concentré, en quantité au moins catalytique;

addition, selon le produit final recherché, de bicarbonate de sodium ou de bicarbonate de potassium en une quantité suffisante pour la précipitation, dans la solution, de l'acide 2-céto-L-gulonique monohydraté non estérifié ainsi que d'impuretés présente, mais non suffisante pour la lactonisation du 2-céto-L-gulonate de méthyle avant que soit atteint l'équilibre de la réaction d'estérification;

séparation du précipité;

addition, selon le produit final recherché, de bicarbonate de sodium ou de bicarbonate de potas-

7

sium, pour la lactonisation du 2-céto-L-gulonate de méthyle; et

séparation du L-ascorbate de sodium ou du L-ascorbate de potassium résultant de la lactonisation, qui se trouve en suspension.

2. Procédé selon la revendication 1, caractérisé en ce que ledit mélange, contenant de l'acide 2-céto-L-gulonique monohydraté, contient en outre du 2-céto-L-gulonate de sodium et en ce que la quantité totale d'acide sulfurique concentré mesurée est telle qu'elle suffit essentiellement également à la conversion du 2-céto-L-gulonate de sodium en acide 2-céto-L-gulonique.

3. Procédé selon la revendication 2, caractérisé en ce que, lors de la conversion du 2-céto-L-gulonate de sodium en acide 2-céto-L-gulonique, le sulfate de sodium formé est séparé par filtration avant l'addition de bicarbonate.

4. Procédé selon la revendication 3, caractérisé en ce que l'on n'utilise d'abord pratiquement que la quantité d'acide sulfurique concentré qui est requise pour la conversion du 2-céto-L-gulonate de sodium en acide 2-céto-L-gulonique, et en ce que l'on n'ajoute la quantité d'acide sulfurique concentré qui catalyse l'estérification qu'après la séparation par filtration du sulfate de sodium formé.

5. Procédé selon l'une des revendications 1 à 4, caractérisé en ce que l'estérification de l'acide 2-céto-gulonique par le méthanol s'effectue en seulement 15 à 90 minutes, selon le degré de la contamination du produit de départ, à 60-70°C, jusqu'à un degré d'estérification de 87 à 91 %.

6. Procédé selon l'une des revendications 1 à 4, caractérisé en ce que, après la première addition de bicarbonate de sodium ou de bicarbonate de potassium, on agite pendant 30-120 minutes le mélange à une température constante, de préférence d'environ 60° C, pour la séparation du précipité.

7. Procédé selon l'une des revendications 1 à 6, caractérisé en ce que l'on refroidit jusqu'à une température dans la plage comprise entre 0 et 5°C le liquide restant après la séparation du précipité et on sépare le 2-céto-L-gulonate de méthyle ainsi précipité.

8. Procédé selon la revendication 7, caractérisé en ce que le bicarbonate de sodium ou bicarbonate de potassium requis pour la lactonisation du 2-céto-L-gulonate de méthyle est ajouté au liquide qui reste après la séparation du précipité et la séparation du 2-céto-L-gulonate de méthyle précipité sous l'effet de l'abaissement de température.

9. Procédé selon la revendication 7, caractérisé en ce que l'on dissout à nouveau dans du méthanol le 2-céto-L-gulonate de méthyle séparé et en ce que l'on ajoute à cette solution le bicarbonate de sodium ou bicarbonate de potassium requis pour la lactonisation du 2-céto-L-gulonate de méthyle.

10. Procédé selon l'une des revendications 1 à 9, caractérisé en ce que le liquide contenant du 2-céto-L-gulonate de méthyle, auquel est ajouté la nouvelle quantité de bicarbonate de sodium ou de potassium aux fins de lactonisation de cet ester, est chauffé pendant 30 à 180 minutes à 60-67°C, après cette addition.

11. Procédé selon l'une des revendications 1 à 10, caractérisé en ce que l'on introduit à nouveau dans le processus le précipité séparé, en tant que produit de départ, au lieu d'acide 2-céto-L-gulonique monohydraté neuf.

12. Procédé selon la revendication 10, caractérisé en ce que l'on réutilise le précipité séparé, au lieu d'acide 2-céto-L-gulonique monohydraté neuf, dans un mélange de départ en solution séparé, et en ce que l'on rejette le précipité formé dans ce mélange de départ en solution.

SCHEMA

```
┌─────────────────────────────────┐                              ┌─────────────────────────────────┐
│ 1.  VERESTERUNG "A"             │                              │     VERESTERUNG "B"             │
│ Ketogulonsäure-Monohydrat       │──────────────────────────┐   │ A                               │
│ Methanol                        │                          └──▶│ Methanol, H₂ SO₄ , Rückfluss   │
│ H₂ SO₄ ,Kochen am Rückfluss     │                              │                                 │
└─────────────────────────────────┘                              └─────────────────────────────────┘
              │                                                                 │
              ▼                                                                 ▼
┌─────────────────────────────────┐                              ┌─────────────────────────────────┐
│ 2.    FÄLLEN "A"                │                              │     FÄLLEN "B"                  │
│ Behandlung der Lösung mit       │                              │ Behandlung mit Bicarbonat       │
│ Bicarbonat                      │                              │ wie "A"/2                       │
└─────────────────────────────────┘                              └─────────────────────────────────┘
              │                                                                 │
              ▼                                                                 ▼
┌─────────────────────────────────┐                              ┌─────────────────────────────────┐
│ 3. FILTRATION "A"/I             │                              │  FILTRATION"B"/I                │
│ Suspension aus 2. abfiltrieren  │                              │      wie "A"/I                  │
└─────────────────────────────────┘                              └─────────────────────────────────┘
         │          │                                                  │              │
         ▼          ▼                                                  ▼              ▼
   ┌──────────┐ ┌──────────────┐                            ┌──────────┐ ┌──────────────┐
   │FILTRAT"A"│ │   FILTER     │────────────┐               │FILTRAT"B"│ │   FILTER     │──┐
   └──────────┘ │ RÜCKSTAND "A"│            │               └──────────┘ │ RÜCKSTAND "B"│  │
        │       └──────────────┘            │                    │       └──────────────┘  ▼
        │                     oder          │                    │    oder
        └───────────────────◀──────────────┘
        │                                                        │
        ▼                                                        ▼
┌─────────────────────────────────┐                  ┌─────────────────────────────────┐
│ 4.  LAKTONISIERUNG "A"          │                  │   LAKTONISIERUNG "B"            │
│ Bicarbonatbehandlung unter      │                  │ Behandlung mit Bicarbonat wie 4.│
│ Rückfluss                       │                  │                                 │
└─────────────────────────────────┘                  └─────────────────────────────────┘
              │                                                    │
              ▼                                                    ▼
┌─────────────────────────────────┐                  ┌─────────────────────────────────┐
│ 5.  FILTRATION "A"/II           │                  │   FILTRATION "B"/II             │
│ Suspension aus 4. abfil-        │                  │ Filtrieren, Waschen wie 5.      │
│ trieren Filterkuchen            │                  │                                 │
│ waschen mit Methanol            │                  │                                 │
└─────────────────────────────────┘                  └─────────────────────────────────┘
              │                                                    │
              ▼                                                    ▼
┌─────────────────────────────────┐                  ┌─────────────────────────────────┐
│ 6.    PRODUKT "A"               │                  │   PRODUKT "B"                   │
│ Ausbeute Min. 90% Na-Asc        │                  │ Ausbeute   Min. 3,5%  Na-Asc    │
│ Reinheit   Min. 99,5%           │                  │ Reinheit   Min. 98%             │
└─────────────────────────────────┘                  └─────────────────────────────────┘
              │                                                    │
              │            ┌─────────────────────┐                │
              └───────────▶│ GESAMT "A"+"B"      │◀───────────────┘
                           │ Ausbeute   93%      │
                           │            Min      │
                           │ Reinheit   99,5%    │
                           │            Min      │
                           └─────────────────────┘
```